# FASCICULE DE BREVET EUROPEEN

(11) **EP 0 925 029 B1**
(45) Date de publication et mention de la délivrance du brevet: **03.09.2003**
(21) Numéro de dépôt: 98936473.2
(22) Date de dépôt: 08.07.1998
(51) Int. Cl.: A61B 17/00

(54) **DISPOSITIF D'EVEINAGE NOTAMMENT POUR ENDOEVEINAGE**
VORRICHTUNG ZUR INSBESONDERE ENDOSKOPISCHEN ENTFERNUNG VON BLUTGEFÄSSEN
STRIPPING DEVICE IN PARTICULAR FOR ENDO-STRIPPING

(30) Priorité: 11.07.1997 FR 9708852
(43) Date de publication de la demande: 30.06.1999
(73) Titulaire: Astra Tech AB, 43121 Mölndal (SE)
(72) Inventeur: Bardeau, Joel, 31840 Seilh (FR); Costacalde, Michael, 82000 Montauban (FR)
(74) Mandataire: Michelet, Alain
(86) Numéro de dépôt international: FR9801466
(87) Numéro de publication internationale: WO99002088

(56) Documents cités:
- AU-A- 6 059 769
- FR-A- 2 619 301
- FR-A- 2 727 617
- US-A- 2 788 787

## Description

La présente invention a pour objet un dispositif d'éveinage chirurgical ou « stripper » dans le vocabulaire anglo-saxon, à usage unique, destiné à l'ablation de veines humaines, notamment lorsque lesdites veines présentent des varices.

De telles opérations sont relativement courantes dans le cas de phlébotomies ou de saphénectomies et elles consistent à arracher la veine malade, la circulation sanguine se rétablissant par des dérivations.

Les dispositifs d'éveinage connus comportent habituellement une tige semi-rigide destinée à être introduite dans la veine à enlever, sur laquelle est ligaturée une extrémité de la veine, l'exérèse étant obtenue par traction sur la tige après ligature d'une extrémité de la veine ou du tronçon. Mais, l'arrachage se traduit par un plissage de la veine à extraire ce qui lèse les tissus adjacents et provoque des saignements et des telangectiasies sur la peau le long du trajet d'extraction.

Les strippers connus ont la caractéristique commune d'être rigides ou semi-rigides pour faciliter la cathétérisation et l'exérèse. Par exemple, FR-A-2 727 617 décrit un dispositif d'éveinage pour réaliser l'extraction d'une veine par invagination, comprenant, à son extrémité distale, un organe d'arrimage. En passant un fil à l'intérieur de la boucle d'arrimage, il est possible selon ce document d'effectuer l'éveinage dans l'un ou l'autre sens. Mais le dispositif consiste toujours en une tige rigide bien que pouvant coulisser dans une gaine.

AU-B-60597 décrit un stripper formé par un câble monofilament orienté dont les extrémités forment des guides facilitant le passage du câble à travers une veine. Une olive de diamètre supérieur à celui des guides peut être montée sur l'un de ceux-ci. Mais le guidage reste aléatoire dans le cas du trajet sinueux des varices.

La présente invention a pour objet de pallier les inconvénients des dispositifs connus et de proposer un dispositif moins traumatisant.

Selon l'invention, le dispositif d'éveinage pour l'ablations d'une veine comprenant une tige semi-rigide est caractérisé en ce que la tige est solidaire à l'une de ses extrémités d'un fil souple, dont la longueur est égale sensiblement à deux fois la longueur de la tige muni, en son milieu, d'un élément d'arrêt.

Le stripper est ainsi constitué par un fil souple et non pas, comme dans la technique antérieure par une tige plus ou moins rigide. Selon l'invention, la tige semi-rigide ne sert qu'à la mise en place du fil avant éveinage. L'élément d'arrêt peut être une bague ou une petite sphère sertie sur le fil.

La tige est, de préférence constituée en une matière plastique biocompatible armée par un mandrin métallique d'environ 1,2 mètre de long raccordé sur un fil souple , par exemple en polyamide, d'environ 2,6 mètres de long sur lequel est placé, sensiblement au milieu, un élément d'arrêt constitué par une bague avantageusement sertie pour la fixation de la veine. La bague d'arrêt ne comporte ni gorge, ni aspérités. Ce sertissage constitue un moyen d'arrêt simple et fiable.

L'invention permet l'éveinage, en particulier des veines saphènes selon les différentes modalités possibles et notamment dans le sens antérograde ou rétrograde, le stripping long ou court, et l'endo ou l'exoéveinage.

De préférence, le stripper est destiné à la réalisation d'exérèse par endoéveinage avec section progressive des collatérales, c'est à dire à l'extraction de la veine malade par invagination ou retournement de la veine sur elle-même à la manière d'un doigt de gant lorsque l'on tire sur la paume de celui-ci. Cette technique présente, par rapport à l'exoéveinage pratiqué à l'origine l'avantage d'être moins traumatisante sur le trajet de la veine. Mais, si, pour une raison quelconque l'endoéveinage a échoué, il est possible de disposer, sur le fil, une olive pour réaliser un exoéveinage c'est à dire une extraction directe sans invagination.

La tige semi-rigide peut présenter, à son extrémité distale, un pliage en queue de cochon qui, comme en soi connu, permet de cathétériser la veine. Elle peut également être droite. Elle n'a pour but que de guider le fil à l'intérieur de la veine. Mais le stripper proprement dit est constitué par le fil qui suit, d'une manière souple le trajet de la veine dans les tissus et constitue le stripper réel pour un endoéveinage.

En outre, la bague est sertie sur le fil et peut servir de point d'arrêt pour une olive adaptable afin de réaliser un exoéveinage.

Le mode opératoire est le suivant: le fil étant mise en place dans la veine, celle-ci est fixée par un point sur la bague d'arrêt et par une traction à partir du point d'incision l'endoéveinage commence et se poursuit jusqu'à ce que toute la veine soit extraite.

Si, la veine se déchire comme cela arrive quelquefois, il n'est pas nécessaire de procéder à une nouvelle cathétérisation. En effet, la longueur du fil permet de réaliser une manoeuvre en sens inverse après avoir fixé la seconde partie de la veine sur le point d'arrêt.

Cette technique peut être mise en oeuvre à partir de la cheville jusqu'à l'aine dans le sens antérograde, le stripping étant effectué de bas en haut en mettant un fil d'arrêt sur le point d'arrêt du fil au niveau de la cheville. Dans ce cas, le point d'arrêt doit cheminer dans la veine jusqu'au niveau de l'aine de façon à effectuer l'endoéveinage de haut en bas ce qui diminue le risque d'atteinte du nerf saphène interne satellite en distalité de la veine.

Il est également possible d'introduire la tige semi-rigide par l'incision de l'aine et de la diriger vers la cheville, l'endoéveinage étant réalisé directement de haut en bas en fixant la veine sur la bague. Dans ce cas, la veine est fixée dès que la bague se présente au niveau de l'aine.

La caractéristique principale du stripper selon l'invention est d'utiliser pour l'éveinage un fil souple qui est mieux adapté pour suivre le trajet sinueux d'une veine qu'une tige même semi-rigide, la tige semi-rigide selon l'invention ne jouant, toutes choses égales par ailleurs, que le rôle d'un guide ou passe-lacet. Il en résulte une meilleure invagination et, en cas de rupture de la veine, il est possible en raison de la longueur du fil de procéder à un endoéveinage en sens inverse sans avoir recours à nouveau à la tige semi-rigide. Enfin, si les manoeuvres de va et vient se traduisent par des échecs répétés, le fil transformé par la mise en place d'une olive au niveau du point d'arrêt permet un exoéveinage.

D'autres caractéristiques et avantages de la présente invention apparaîtront au cours de la description qui va suivre d'un exemple de réalisation particulier donné uniquement à titre d'exemple non limitatif en regard des figures qui représentent :
- La figure 1, une vue d'un stripper selon l'invention;
- Les figures 2, 2a et 2b, des vues de l'olive pouvant être rapportée sur le fil.

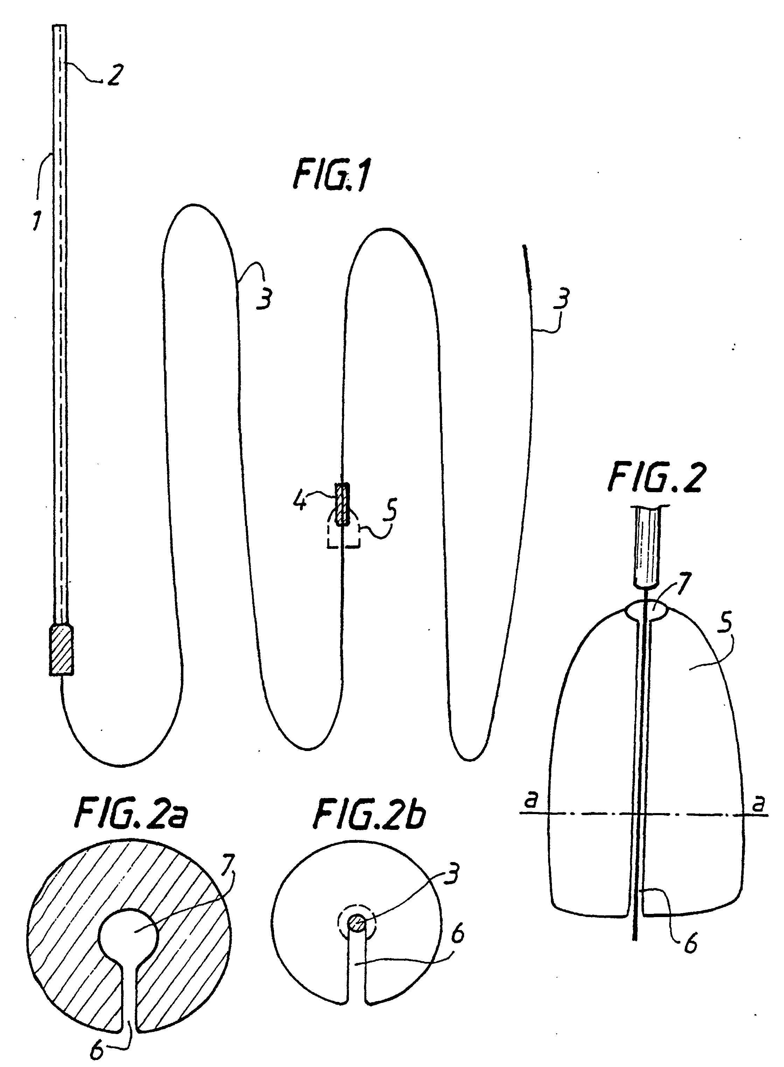

Sur la figure 1, on voit que le stripper se compose d'une tige semi-rigide 1 qui peut être terminée à sa partie avant 2 par une déformation en queue de cochon qui dans certains cas facilite la pénétration de la tige dans le trajet parfois sinueux de la veine et peut éviter des déchirures de celle-ci. Mais l'extrémité avant peut être rectiligne. La tige a une longueur de l'ordre de 1,2 mètre de manière à ce que son extrémité avant 2 puisse parcourir toute la longueur d'une veine. Sur la partie arrière de la tige 1 est serti un fil souple 3 dont la longueur est sensiblement égale à deux fois la longueur de la tige 1, c'est à dire deux fois la longueur possible d'une veine. Dans le milieu du fil 3 est sertie une bague 4 destinée à servir de point d'attache ou de ligature de la veine à extraire.

Comme indiqué précédemment, le stripper peut être utilisé dans le sens antérograde ou dans le sens rétrograde.

Les figures 2 à 2b représentent une olive 5, du type de celles qui sont utilisées dans les articles de pêche, pouvant être montée très simplement sur la bague 4 dans le cas où un exoéveinage s'avère nécessaire. L'olive présente une fente radiale 6 dans laquelle peut être introduit le fil 3 comme cela est représenté sur la figure 2b qui est une vue par dessous de l'olive 5. Au niveau a dont la coupe transversale est représentée sur la figure 2a, la fente 6 débouche dans un alésage central 7 dont le diamètre intérieur est légèrement inférieur au diamètre extérieur de la bague d'arrêt 4. L'olive peut ainsi être montée en force sur la bague ce qui assure la solidarisation souhaitée.

Il va de soi que de nombreuses variantes peuvent être apportées, notamment par substitution de moyens techniques équivalents, sans sortir pour cela du cadre de l'invention, tel que défini par les revendications.

## Revendications

1. Dispositif d'éveinage pour l'ablation d'une veine par invagination, comprenant une tige semi-rigide (1), **caractérisé en ce que** la tige (1) est solidaire à l'une de ses extrémités (2) d'un fil souple (3) dont la longueur est au moins sensiblement le double de celle de la tige (1), et comprenant un élément d'arrêt (4) sur le fil souple (3) à une distance de la tige (1) au moins environ égale à la longueur de ladite tige (1).

2. Dispositif selon la revendication 1 **caractérisé en ce que** le fil souple (3) est en polyamide.

3. Dispositif selon la revendication 1 ou 2, **caractérisé en ce qu'**il comprend une olive (5) attachée au fil (3) au niveau de l'élément d'arrêt (4).

4. Dispositif selon la revendication 3, **caractérisé en ce qu'**il comprend, de plus, une olive (5) présentant, un trou axial (7) et une fente radiale (6) dont la largeur est sensiblement égale au diamètre du fil (3), le trou axial débouchant à sa partie supérieure dans un alésage (7) dont le diamètre intérieur est inférieur au diamètre de l'élément d'arrêt (4).

## Claims

1. Device for removing blood vessels comprising a semi-rigid rod, wherein the rod (1) is secured at one of its ends (2) to a flexible thread (3) which length is at least about twice as long as the rod (1) and comprising a stop element (4) on the flexible thread (3), located on the thread (3), at a distance from the semi-rigid rod (1), at least about equaling to the length of said rod (1).

2. Device for removing blood vessels according to claim 1 wherein said flexible thread (3) is made of polyamide.

3. Device for removing blood vessels according to any of claim 1 or 2 wherein the device comprises a traveller (5) attached to the thread (3) at the stop element (4).

4. Device for removing blood vessels according to claim 3 comprising a traveller (5) with an axial hole (7) and a radial slit (6), said slit having a width equal to about the thread (3) diameter, and said axial hole (7) opening at its top into a bore (7) having an internal diameter inferior to the stop element (4) external diameter.

## Patentansprüche

1. Vorrichtung zum Entfernen einer Vene mittels Einführens eines Objekts, mit einem halbstarren Stab, **dadurch gekennzeichnet, daß** der Stab (1) an einem seiner Enden (2) mit einem beweglichen Faden (3) fest verbunden ist, dessen Länge im Wesentlichen das Doppelte der Länge des Stabes (1) ist und der auf dem beweglichen Faden (3) in einer Entfernung vom Stab (1), die wenigstens gleich der Länge des Stabes (1) ist, ein Arretierungsmittel (4) aufweist.

2. Vorrichtung nach Ansprch 1, **dadurch gekennzeichnet, daß** der bewegliche Faden (3) aus einem Polyamid ist.

3. Vorrichtung nach Anspruch 1 oder 2, **dadurch gekennzeichnet, daß** sie eine Olive (5) aufweist, die in Höhe des Arretierungsmittels (4) am beweglichen Faden (3) befestig ist.

4. Vorrichtung nach Anspruch 3, **dadurch gekennzeichnet, daß** sie eine Olive (5) mit einem axialen Loch (7) und einem radialen Schlitz (6), dessen Weite im Wesentlichen gleich dem Durchmesser des Fadens (3) ist, aufweist, wobei das axiale Loch (7) an dessen oberen Ende in eine Bohrung (7) einmündet, deren innerer Durchmesser kleiner als der Durchmesser des Arretierungsmittels (4) ist.
